# EUROPÄISCHE PATENTSCHRIFT

(11) **EP 0 850 630 B1**
(45) Veröffentlichungstag und Bekanntmachung des Hinweises auf die Patenterteilung: **12.03.2003**
(21) Anmeldenummer: 97122574.3
(22) Anmeldetag: 19.12.1997
(51) Int. Cl.: A61F 13/15

(54) **Saugfähiges Produkt für Ausscheidungen des menschlichen Körpers**
Absorbant product for excreta of the human body
Produit absorbant pour excréments humains

(30) Priorität: 23.12.1996 DE 19654056
(43) Veröffentlichungstag der Anmeldung: 01.07.1998
(73) Patentinhaber: W. Pelz GmbH & Co., 23812 Wahlstedt/Holstein (DE)
(72) Erfinder:
(74) Vertreter: Patentanwälte Hauck, Graalfs, Wehnert, Döring, Siemons, Schildberg

(56) Entgegenhaltungen:
- EP-A- 0 189 102
- EP-A- 0 531 714
- EP-A- 0 540 041
- EP-A- 0 625 602
- DE-A- 3 620 077
- DE-A- 3 705 244

## Beschreibung

Die Erfindung bezieht sich auf ein saugfähiges Produkt für Ausscheidungen des menschlichen Körpers. Das saugfähige Produkt kann insbesondere eine Slipeinlage, Damenbinde oder eine, Schweißeinlage sein.

Es sind bereits Slipeinlagen bekannt, deren Saugkissen aus geprägtem Wattematerial, insbesondere Fasern aus Viskose und/oder gebleichter Baumwolle besteht, das als Vlies ausgebildet sein kann (vgl. DE 36 20 077 C2, EP-A-0 625 602, EP-A-0 189 102, EP-A-0 540 041). Diese Saugkissen haben die Eigenschaft zu flusen. Außerdem sind Slipeinlagen auf dem Markt, deren Saugkissen aus einem Airlaid besteht, d.h. aus einem in einem Luftstrom geformten und mit einem Bindemittel oder thermisch verfestigten Cellulosematerial. Diese Saugkissen sind aufwendig in der Herstellung, teuer und fühlen sich auf der Haut unangenehm an. Außerdem ist der Herstellungsabfall in der Produktion nicht verwertbar. Die beiden bekannten Slipeinlagen haben deshalb eine die Abgabe von Flusen behindernde bzw. den Tragecomfort verbessernde Umhüllung aus einem flüssigkeitsdurchlässigen Material. Die Umhüllung steigert jedoch den Herstellungsaufwand und damit die Kosten.

Davon ausgehend liegt der Erfindung die Aufgabe zugrunde, ein saugfähiges Produkt für Ausscheidungen des menschlichen Körpers zu schaffen, das weniger flust, ein angenehmeres Tragegefühl vermittelt und den Produktionsaufwand bzw. die Herstellungskosten mindern kann.

Die Aufgabe wird durch saugfähige Produkte mit den Merkmalen des Anspruchs 1 gelöst. Vorteilhafte Ausgestaltungen der Produkte sind in den Unteransprüchen angegeben.

Die erfindungsgemäßen Produkte basieren auf einem wasservernadelten Faservliesstoff. Das Prinzip der Wasservernadelung (im Englischen als "Hydraulic-" oder "Hydro entanglement", Waterjet entanglement", Spunlacing" oder "Spunlaying" bekannt) besteht darin, eine Faserverwirbelung und damit eine Verfestigung in einer Vliesbahn durch Verwendung der durch Wasserstrahlen beförderten kinematischen Energie zu erreichen. In der Praxis wird die Wasservernadelung durchgeführt, indem mittels einer Düsengruppe eine Vielzahl feinster Wasserstrahlen hoher Energie auf eine durchlaufende Vliesbahn gerichtet wird. Dabei geht die Verfestigung um so tiefer in das Material, je höher die Strahlenergie ist. Die Vliesbahn befindet sich auf einem Sieb, durch das das Wasser abgezogen wird. Auch kann das Sieb durch Reflektion der Wasserstrahlen eine zusätzliche Verwirbelung bewirken. Die Verwirbelung kann in einem oder mehreren Stufen mit einer oder mehreren Gruppen quer zur Laufrichtung nebeneinander angeordneter Düsen (Düsenbalken) erfolgen.

Bei dem einen erfindungsgemäßen Produkt, das als Dünnbinde, Slipeinlage oder Schweißeinlage ausgebildet sein kann, ist ein Saugkissen bzw. ein Ausgangsmaterial für seine Herstellung durch Wasservernadelung behandelt, so daß es an der körperzugewandten Seite und in der Tiefe mehr oder weniger verwirbelte Fasern hat und entsprechend verfestigt ist. Hierdurch wird erreicht, daß das Faservlies im Gegensatz zu einem nicht wasservernadelten Faservlies nicht mehr flust. Außerdem fühlt sich der wasservernadelte Faservliesstoff wesentlich weicher und anschmiegsamer an und hat einen textilen Charakter, so daß es sich anfühlt wie ein Baumwollslip. Eine Umhüllung kann entfallen, wodurch bei vereinfachtem Aufbau ein verbessertes saugfähiges Produkt erreicht wird. Das Saugkissen hat jedoch eine Wäscheschutzschicht an der körperabgewandten Seite, die einen Flüssigkeitsaustritt und ein Verschmutzen der Wäsche verhindert.

Das Saugkissen des Produktes wird an der körperzugewandten Seite vernadelt, wobei ein Flusen an der körperzugewandten Seite durch die Wäscheschutzschicht behindert werden kann. Das Saugkissen kann jedoch auch an der körperabgewandten Seite wasservernadelt sein. Auch eine Wasservernadelung nur an der körperabgewandten Seite kommt in Betracht, weil hierdurch zumindest ein Flusen an dieser Seite verhindert und eine Verfestigung erreicht wird. Das zwischen körperzugewandter und körperabgewandter Seite liegende Fasermaterial kann im Hinblick auf eine große Aufnahmefähigkeit für Körperflüssigkeit und die Weichheit des Produktes unverfestigt bleiben. Das Produkt kann jedoch auch über die Tiefe unterschiedliche Grade der Verfestigung durch Wasservernadeln haben. So kann es beispielsweise in dem Bereich der körperabgewandten Oberfläche stärker als im Bereich der körperzugewandten Oberfläche vernadelt sein, um an der körperabgewandten Seite Formfestigkeit bzw. Flüssigkeitsdichtigkeit zu fördern und an der körperzugewandten Seite lediglich das Flusen in ausreichendem Maße zu behindern, ohne das Produkt dort zu stark zu verfestigen.

Der Faservliesstoff kann ausschließlich aus Baumwollfasern bestehen oder eine Mischung von Baumwollfasern mit Viskose oder mit synthetischen Fasern, insbesondere thermisch verfestigbaren Fasern, z.B. Polypropylen-, Polyethylen- oder Bikomponentenfasern enthalten. Hierdurch können insbesondere die Absorptions-, Festigkeits- und taktilen Eigenschaften des Produktes gesteuert werden. Die thermisch verfestigbaren Fasern können durch Wärmebehandlung zu einer mehr oder weniger innigen Verbindung gebracht werden. Insbesondere werden Mischungen aus etwa 85 % Baumwollfasern und etwa 15 % thermisch verfestigbaren Fasern in Betracht gezogen.

Die hier einsetzbaren Baumwollfasern können im Unterschied zu natürlichen Baumwollfasern Flüssigkeit anlagern und/oder aufsaugen. Diese Fähigkeit kann den Baumwollfasern insbesondere durch eine Bleichbehandlung verliehen worden sein. Bei Baumwollfasern oder dgl. Naturfasern kann auch durch anderweitige Behandlung ein wasserabstoßender Wachs- oder Fettfilm von den Fasern entfernt werden. In Betracht kommt beispielsweise eine Behandlung mit Wasserstrahlen hoher Energie, welche die Fettschicht ablöst (vgl. EP 0 456 795 B1).

In das Saugkissen kann eine Schicht aus hydrophobem Material integriert sein, insbesondere in Form eines gitterartigen Vlieses. Geeignete gitterartige Vliese aus synthetischem Material sind auf dem Markt erhältlich. Die integrierte Schicht bewirkt eine Verteilung der Flüssigkeit von der Auftropfstelle über das Saugkissen. Ferner kann in das Saugkissen ein superabsorbierendes Material (z.B. in Faser-, Pulver- oder Granulatform) integriert sein, welches die Aufnahmekapazität beträchtlich erhöht. Dieses kann eine Verteilung mit unterschiedlichen Konzentrationen in den verschiedenen Richtungen des Saugkissens haben.

Zum Behindern einer seitlichen Flusenabgabe kann das Saugkissen am Umfang eine Randprägung aufweisen. An der körperzugewandten Seite kann das Saugkissen Musterprägungen haben, die ebenfalls der Flüssigkeitsverteilung oder Stabilisierung dienen können. Diese gehen vorzugsweise mehr oder weniger durch das Saugkissen, also maximal von der körperzugewandten bis zur körperabgewandten Seite und verfestigen einen inneren Bereich des Saugkissens, der weniger oder nicht wasservernadelt ist. Die Musterprägungen bzw. Randprägungen verhindern, daß sich die verschiedenen Schichten des Faservliesstoffes, insbesondere die körperzugewandte Schicht und die darunterliegende, flüssigkeitsspeichernde Schicht, voneinander lösen, Fasern von den Randbereichen abgehen und die Aufnahmekapazität des Saugkissens lokal überschritten wird und Flüssigkeit in den Randbereichen austritt.

Die Wäscheschutzschicht an der körperabgewandten Seite des Saugkissens kann eine flüssigkeitsundurchlässige Folie sein, beispielsweise aus einem Kunststoff, insbesondere aus einem Polyolefin. Dabei kann es sich aber auch um einen hydrophoben Vliesstoff handeln. Dieser kann durch Wasservernadelung oder durch Klebung mit dem Saugkissen verbunden sein. Das erhöht ebenfalls die Stabilität. Ferner kann es sich bei der Wäscheschutzschicht um eine besondere Schicht des Faservliesstoffes handeln, die reich an hydrophoben Fasern ist, beispielsweise thermisch verfestigbaren Fasern oder Naturfasern mit einer vorhandenen Wachs- oder Fettschicht.

Auch auf der körperzugewandten Seite des Saugkissens kann ein hydrophober Vliesstoff angeordnet sein, um eine Rücknässung der körperzugewandten Seite zu verhindern. Dieser kann ebenfalls durch Wasservernadelung oder durch Klebung mit dem Saugkissen verbunden sein. Die Klebung zur Befestigung des hydrophoben Vliesstoffes an der körperzugewandten bzw. körperabgewandten Seite kann durch einen aufgesprühten, einen linien-, schuppen- oder flächenförmig aufgetragenen Heißleim erfolgen. Der Vliesstoff für die körperzugewandte bzw. körperabgewandte Seite kann aus Polyester-, Polypropylen-, Bikomponentenfasern und/oder einem anderen thermisch verfestigbaren Fasermaterial bzw. deren Mischungen bestehen. Es kann sich auch um ein Spinnvlies handeln, ein thermoverfestigtes kardiertes Vlies oder ein aerodynamisch verfestigtes Vlies.

Schließlich kann die Wäscheschutzschicht an der körperabgewandten Seite mit mindestens einer Haftleimfläche versehen sein. Diese kann wiederum an der körperabgewandten Seite eine Silikonabdeckung tragen, nach deren Ablösung das Produkt an der Wäsche der Benutzerin befestigt werden kann.

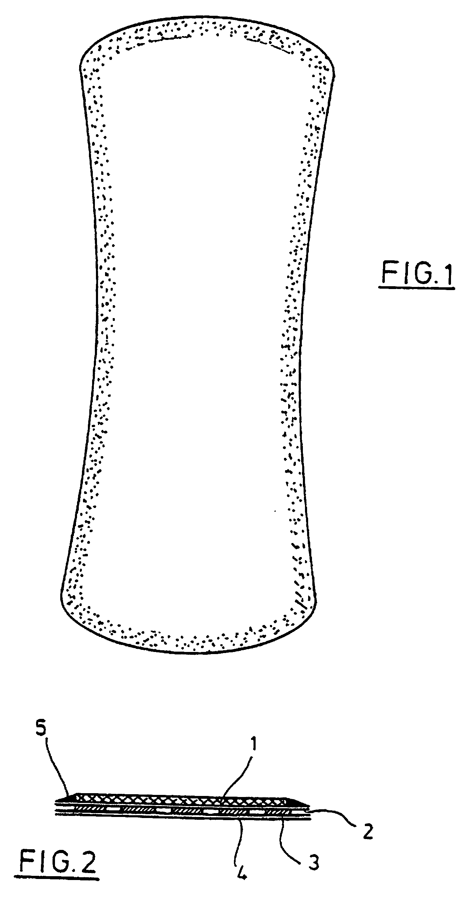

Ein Ausführungsbeispiel einer Slipeinlage ist in der anliegenden Zeichnung in der Draufsicht (fig. 1) und im Querschnitt (Fig. 2) gezeigt.

Demnach hat die Slipeinlage an der körperzugewandten Seite ein Saugkissen 1 aus einem wasservernadelten Faservliesstoff (Spunlace-Vliesstoff) mit einem Flächengewicht von 120 g/m².

An der körperabgewandten Seite des Saugkissens 1 ist eine Wäscheschutzfolie 2 oder ein hydrophober Vliesstoff als Wäscheschutz angebracht.

An der körperabgewandten Seite der Wäscheschutzfolie 2 sind mehrere parallele Haftleimstreifen 3 angeordnet, die in Längsrichtung der Slipeinlage verlaufen.

Die Haftleimstreifen 3 sind wiederum durch ein zusammenhängendes Silikonpapier 4 abgedeckt, nach dessen Entfernen die Einlage im Steg eines Höschen befestigt werden kann.

Das Saugkissen 1 hat am Umfang eine umlaufende Randprägung 5, die den seitlichen Austritt von Fasern und Flüssigkeit behindert.

Die Slipeinlage ist in der Zeichnung etwa in Originalgröße dargestellt. Der Querschnitt ist durch die verdickten Endbereiche der in der Draufsicht hantelförmigen Slipeinlage gelegt.

## Patentansprüche

1. Saugfähiges Produkt für Ausscheidungen des menschlichen Körpers, nämlich Dünnbinde, Slipeinlage oder Schweißeinlage, dessen körperzugewandte Seite von der körperzugewandten, wasservernadelten Seite eines Faservliesstoffes gebildet ist und das Saugkissen (1) bildet, mit einer Wäscheschutzschicht (2) an der körperabgewandten Seite des Saugkissens (1) und Musterprägungen an der körperzugewandten Seite und/oder einer Randprägung am Umfang des Saugkissens (1) zur Stabilisierung des Saugkissens (1), **dadurch gekennzeichnet daß** der zwischen körperzugewandter und körperabgewandter Seite angeordnete Faservliesstoff weniger der nicht wasservernadelt ist.

2. Produkt nach Anspruch 1 mit einem baumwollfasern enthaltenden wasservemadelten Faservliesstoff.

3. Produkt nach Anspruch 2 mit einem wasservemadelten Faservliesstoff, der gebleichte Baumwollfasern enthält.

4. Produkt nach Anspruch 2 oder 3 mit einem eine Mischung von Baumwollfasern mit Viskose oder mit thermisch verfestigbaren Fasern enthaltenden wasservernadelten Faservliesstoff.

5. Produkt nach Anspruch 4 mit einem eine Mischung von 75 bis 85 % Baumwollfasern und 25 bis 5 % synthetische Fasern enthaltenden wasservernadelten Faservliesstoff.

6. Produkt nach Anspruch 5 mit einem etwa 85 % Baumwollfasern und etwa 15 % synthetische Fasern enthaltenden wasservemadelten Faservliesstoff.

7. Produkt nach einem der Ansprüche 1 bis 6 mit einem an der körperabgewandten Seite wasservernadelten Faservliesstoff.

8. Produkt nach einem der Ansprüche 1 bis 7 mit einem in Tiefenrichtung in unterschiedlichem Ausmaß wasservernadelten Faservliesstoff.

9. Produkt nach einem der Ansprüche 1 bis 8 mit einer in das Saugkissen integrierten Schicht aus hydrophobem Material.

10. Produkt nach Anspruch 9 mit einer als gitterartigem Vlies ausgebildeten integrierten Schicht.

11. Produkt nach einem der Ansprüche 1 bis 10 mit einem in das Saugkissen integrierten superabsorbierenden Material, insbesondere in Faser-, Pulver- oder Granulatform.

12. Produkt nach Anspruch 11, bei dem das superabsorbierende Material als Saugkern in das Saugkissen integriert ist.

13. Produkt nach einem der Ansprüche 1 bis 12 mit einem wasservemadelten Faservliesstoff mit einem Flächengewicht von 60 bis 300 g/m².

14. Produkt nach Anspruch 13 mit einem wasservernadelten Faservliesstoff mit einem Flächengewicht von etwa 120 g/m².

15. Produkt nach Anspruch 13 mit einem wasservernadelten Faservliesstoff mit einem Flächengewicht von etwa 250 g/m².

16. Produkt nach einem der Ansprüche 1 bis 15 mit einem an der körperabgewandten Seite zu einer Wäscheschutzschicht (2) wasservernadelten Faservliesstoff.

17. Produkt nach einem der Ansprüche 1 bis 16 mit einer flüssigkeitsundurchlässigen Folie als Wäscheschutzschicht (2).

18. Produkt nach einem der Ansprüche 1 bis 17 mit einem hydrophoben Vliesstoff als Wäscheschutzschicht (2) an der körperabgewandten Seite des Saugkissens (1).

19. Produkt nach Anspruch 18 mit einem Polyestervlies, Polypropylenvlies, Polyethylenvlies oder anderem synthetischen Vlies als hydrophobem Vliesstoff.

20. Produkt nach Anspruch 18 oder 19 mit einem hydrophoben Vliesstoff mit einem Flächengewicht von 12 bis 24 g/m², vorzugsweise etwa 16 g/m².

21. Produkt nach einem der Ansprüche 17 bis 20 mit einem mit dem Saugkissen (1) vernadelten hydrophoben Vliesstoff.

22. Produkt nach einem der Ansprüche 17 bis 21 mit einem mit dem Saugkissen (1) versiegelten hydrophoben Vliesstoff.

23. Produkt nach einem der Ansprüche 1 bis 22 mit einer an hydrophoben Fasern reichen Schicht an der körperabgewandten Seite des Faservliesstoffes als Wäscheschutzschicht (2).

24. Produkt nach einem der Ansprüche 1 bis 23 mit mindestens einer Haftleimfläche (3) an der körperabgewandten Seite der Wäscheschutzschicht (2).

25. Produkt nach Anspruch 24 mit einer Silikonpapier-Abdeckung (4) an der körperabgewandten Seite der Haftleimfläche (3).

## Claims

1. An absorptive product for excreta of the human body, namely a thin sanitary napkin, pad for briefs or anti-transpiration pad whose side facing the body is formed by the proximal, waterjet entangled side of a non-woven fabric and forms the absorbent cushion (1), including an underwear protective layer (2) on the distal side of the absorbent cushion (1) and embossed patterns on the proximal side and/or an embossed bordering at the periphery of the absorbent cushion (I) to stabilize the absorbent cushion (1), **characterized in that** the non-woven fabric disposed between the proximal and distal sides is less or not waterjet entangled.

2. The product as claimed in claim 1, comprising a waterjet entangled non-woven fabric containing cotton fibres.

3. The product as claimed in claim 2, comprising a waterjet entangled non-woven fabric containing bleached cotton fibres.

4. The product as claimed in claim 2 or 3, comprising a waterjet entangled non-woven fabric containing a blend of cotton fibres with rayon fibres or thermally bondable fibres.

5. The product as claimed in claim 4, comprising a waterjet entangled non-woven fabric containing a blend of from 75 to 85 % of cotton fibres and from 25 to 5 % of man-made fibres.

6. The product as claimed in claim 5, comprising a waterjet entangled non-woven fabric containing about 85 % of cotton fibres and about 15 % of man-made fibres.

7. The product as claimed in any one of claims 1 to 6, comprising a non-woven fabric waterjet entangled on the proximal side.

8. The product as claimed in any one of claims 1 to 7, comprising a non-woven fabric waterjet entangled to a different degree in a depth direction.

9. The product as claimed in any one of claims 1 to 8, comprising a layer of hydrophobic material integrated in the absorbent cushion.

10. The product as claimed in claim 9, comprising an integrated layer configured as a lattice-like fibre web.

11. The product as claimed in any one of claims 1 to 10, comprising a super-absorbent material integrated in the absorbent cushion, particularly of a fibrous, powdered or granular shape.

12. The product as claimed in claim 11 wherein the super-absorbent material is integrated in the absorbent cushion as an absorptive core.

13. The product as claimed in any one of claims 1 to 12, comprising a waterjet entangled non-woven fabric having a basic weight of from 60 to 300 g/m².

14. The product as claimed in claim 13, comprising a waterjet entangled non-woven fabric having a basic weight of about 120 g/m².

15. The product as claimed in claim 13, comprising a waterjet entangled non-woven fabric having a basic weight of about 250 g/m².

16. The product as claimed in any one of claims 1 to 15, comprising a non-woven fabric waterjet entangled on the distal side to form an underwear protective layer (2).

17. The product as claimed in any one of claims 1 to 16, comprising a sheeting impermeable to liquid as an underwear protective layer (2).

18. The product as claimed in any one of claims 1 to 17, comprising a hydrophobic non-woven fabric as an underwear protective layer (2) on the distal side of the absorbent cushion.

19. The product as claimed in claim 18, comprising a polyester web, polyethylene web or another man-made fibre web as a hydrophobic non-woven fabric.

20. The product as claimed in claim 18 or 19, comprising a hydrophobic non-woven fabric having a basic weight of from 12 to 24 g/m², preferably about 16 g/m².

21. The product as claimed in any one of claims 17 to 20, comprising a hydrophobic non-woven fabric waterjet entangled with the absorbent cushion (1).

22. The product as claimed in any one of claims 17 to 21, comprising a hydrophobic non-woven fabric sealed with the absorbent cushion (1).

23. The product as claimed in any one of claims 1 to 22, comprising a layer rich in hydrophobic fibres on the distal side of the underwear protective layer (2).

24. The product as claimed in any one of claims 1 to 23, comprising at least one layer of pressure-sensitive adhesive (3) on the distal side of the underwear protective layer (2).

25. The product as claimed in claim 24, comprising a silicone paper cover (4) on the distal side of the layer of pressure-sensitive adhesive (3).

## Revendications

1. Produit absorbant pour des excréments du corps humain, c'est-à-dire une bande hygiénique mince, un protège-dessous ou une pièce anti-transpiration, le coté dirigé vers le corps duquel est constitué par le coté dirigé vers le corps d'un non tissé aiguilleté hydrauliquement et qui forme le coussin absorbant (1), avec une couche protectrice pour le linge (2) dans le coté détourné du corps du coussin absorbant (1) et avec des gaufrages de dessin dans le coté dirigé vers le corps et/ou un gaufrage de pourtour dans le périmètre du coussin absorbant pour la stabilisation du coussin absorbant, characterisé en ce que le non tissé disposé entre le coté dirigé vers le corps et le coté détourné du corps est moins ou pas du tout aiguilleté hydrauliquement.

2. Produit selon la revendication 1, avec un non tissé aiguilleté hydrauliquement comprenant des fibres de coton.

3. Produit selon la revendication 2 avec un non tissé aiguilleté hydrauliquement comprenant des fibres de coton décolorées.

4. Produit selon la revendication 2 ou 3 avec un non tissé aiguilleté hydrauliquement, ledit non tissé comprenant un mélange de fibres de coton avec viscose ou avec des fibres thermiquement solidifiables.

5. Produit selon la revendication 4 avec un non tissé aiguilleté hydrauliquement, ledit non tissé comprenant un mélange de 75 à 85% par poids de fibres de coton et 25 à 5% par poids de fibres synthétiques.

6. Produit selon la revendication 5 avec un non tissé aiguilleté hydrauliquement, ledit non tissé comprenant 85% par poids de fibres de coton environ et 15% par poids de fibres synthétiques environ.

7. Produit selon une des revendications 1 à 6 avec un non tissé qui est aiguilleté hydrauliquement sur le coté détourné du corps.

8. Produit selon une des revendications 1 à 7 avec un non tissé aiguilleté dans une mesure inégale dans la direction vers l'intérieur.

9. Produit selon une des revendications 1 à 8 avec une couche de produit hydrophobe integrée dans le cuisson absorbant.

10. Produit selon la revendication 9 avec une couche intégrée réalisée comme non tissé en forme de grillage.

11. Produit selon une des revendications 1 à 10 avec du matériau super-absorbant intégré dans le cuisson absorbant, notamment en forme de fibre, de poudre ou granulé.

12. Produit selon la revendication 11, le matériau super-absorbant étant intégré comme centre d'absorption dans le cuisson absorbant.

13. Produit selon une des revendications 1 à 12 avec un non tissé aiguilleté hydrauliquement, ledit non tissé ayant un poids de base de 60 à 300 g/m².

14. Produit selon la revendication 13 avec un non tissé aiguilleté hydrauliquement, ledit non tissé ayant un poids de base de 120 g/m² environ.

15. Produit selon la revendication 13 avec un non tissé aiguilleté hydrauliquement, ledit non tissé ayant un poids de base de 250 g/m² environ.

16. Produit selon une des revendications 1 à 15, avec un non tissé, ledit non tissé étant aiguilleté hydrauliquement pour former une couche protectrice pour le linge dans le coté détourné du corps.(2)

17. Produit selon une des revendications 1 à 16, avec une feuille imperméable pour des liquides comme couche protectrice pour le linge.

18. Produit selon une des revendications 1 à 17, avec un non tissé hydrophobique comme couche protectrice pour le linge (2) dans le coté détourné du corps du coussin absorbant.

19. Produit selon la revendication 18 avec un non tissé de polyester, un non tissé de polypropylène, un non tissé de polyéthylène ou un autre non tissé synthétique comme le non tissé hydrophobique.

20. Produit selon la revendication 18 ou 19 avec un non tissé hydrophobique, ledit non tissé ayant un poids de base de 12 à 24 g/cm², préférablement 16 g/m² environ.

21. Produit selon une des revendications 17 à 20, avec un non tissé hydrophobique qui est aiguilleté avec le coussin absorbant (1).

22. Produit selon une des revendications 17 à 21, avec un non tissé hydrophobique scellé avec le coussin absorbant (1);

23. Produit selon une des revendications 1 à 22, avec une couche riche en fibres hydrophobiques dans le coté détourné du corps du non tissé comme couche protectrice pour le linge (2).

24. Produit selon une des revendications 1 à 23, avec au moins un pan de colle adhésive (3) dans le coté détourné du corps de la couche protectrice (2).

25. Produit selon la revendication 24 avec une couverture de papier silicone (4) dans le coté détourné du pan de colle adhésive (3).
